# EUROPEAN PATENT APPLICATION

(11) **EP 1 679 082 A1**
(43) Date of publication of application: **12.07.2006**
(21) Application number: 05000276.5
(22) Date of filing: 07.01.2005
(51) Int. Cl.: A61K 49/00

(54) **Use of cyanine dyes for the diagnosis of proliferative diseases**

(71) Applicant: Schering AG, 13342 Berlin (DE)
(72) Inventor: Licha, Kai, 14612 Falkensee (DE); Schirner, Michael, 13158 Berlin (DE); Pessel, Martin, 10437 Berlin (DE); Bahner, Malte, 10781 Berlin (DE)
(74) Representative: Krauss, Jan

(57) **Abstract**

The present invention concerns the use of the cyanine dye SF64 for the diagnosis of proliferative diseases upon administration of less than 5 mg/kg body weight.

## Description

The present invention concerns the use of the cyanine SF64 for the diagnosis of proliferative diseases, in particular tumor diseases upon administration of less than 0.1 mg/kg body weight.

### Background of the invention

Cancer is the second leading cause of death among Americans and is responsible for one out of every four deaths in the United States. In 2004 over 560,000 Americans or more than 1,500 people a day will die of cancer. Over 18 million new cases of cancer have been diagnosed since 1990 and about 1.4 million new cases will be diagnosed in 2004 alone. This estimate does not include preinvasive cancer or more than 1 million cases of none-melanoma-skin cancer expected to be diagnosed this year. The financial costs of cancer are overwhelming. According to the National Institute of Health cancers cost in the United States are more than $ 189 billion in 2003. This amount includes over $ 64 billion in direct medical costs and more than $ 125 billion in lost productivity. The number of new cancer fatalities could be reduced substantially by early diagnosis of cancers. Therefore, cancer screening in particular screening tests for breast, cervical and colorectal cancers could significantly reduce the number of deaths from this diseases by finding them early when they are most treatable. Screening tests for breast, cervical and colorectal cancers may actually prevent these cancers from ever developing by detecting treatable pre-cancerous conditions.

Ideally screening methods are sensitive and specific, can be performed rapidly, are none-invasive, cheap and are associated with no or only neglectable side effects. For the screening for, e.g. mammary tumors the currently established screening standard involves x-ray imaging of the breast tissue a procedure which is also called mammography. Other methods involve nuclear magnetic resonance imaging, ultrasonography and thermography. By far the most widely administered screening method is x-ray mammography, which has a high specificity (about 80%), however, the sensitivity depends largely on the interpretation of imaging data by the radiologist. The spatial resolution of mammography is low and tumors detected usually have a size of 1 cm or larger. However, mammography has been associated with a significant and cumulative risk of radiation exposure in particular in premenopausal woman, which have denser breast tissue and require higher radiation dosages as older woman to obtain a sufficient sensitivity. Mammography has also been criticized for the forceful manipulation of the breast during the procedure, which might facilitate dissemination of tumor cells. Magnetic resonance imaging (MRI) has been used increasingly in the past in particular after a tumor had been identified with a different method. MRI imaging due to its high spatial resolution has a vastly superior sensitivity in comparison to x-ray based imaging techniques like mammography, however, it is less specific (specificity ranging from 37% to 97% and the predictive value for woman not previously diagnosed with breast cancer is less than 2%) much more expensive and time consuming and, thus, less amenable to mass screening of patients.

Recently, a further method called diffuse optical tomography (DOT) in the near-infrared (NIR) has emerged as a new imaging method with a high potential in a variety of medical imaging applications. This technique has the capacity to produce quantitative images of intrinsic and extrinsic absorption and scattering (Arridge, S. R. (1995) Appl. Opt. 34: 7395-7409 and Gonatas, C. P. *et al.* (1995) *Phys. Rev. E.* 52: 4361-4365). Ntziachristos, *V. et al.* (2000) *Proc. Nat. Aca. Sci. US.A.* 97: 2767-2772) describe the use of indocyanine green (ICG) for contrast enhancement during optical imaging of the human *breast in vivo.* Optical imaging of large organs such as breast is often feasible because of the low absorption of tissue in the 700 to 850 nm spectral region. In fact, light has been investigated since the late 1920s as a diagnostic tool for breast cancer by transillumination. Transillumination, however, had low spatial resolution and afforded little in spectral quantification of the lesions detected. Hence, transillumination did not attain sufficient sensitivity and specificity to be used clinically. Vast improvements in the mathematical modelling of light propagation in tissue combined with technological advances have now made possible the application of tomographic principles for imaging with diffuse light. Diffuse optical tomography has dramatically improved the ability to localize and quantify tissue structures with light. Furthermore, the method employs none-ionizing radiation and uses relatively low costs instrumentation, which makes it suitable for mass screening of breast or other cancers accessible by light. Fluoro-chromes like, e.g. indocyanine (ICG, which is an absorber and fluorophor in the NIR) have been used as contrast agents. For DOT using FCG as contrast agent it has been reported that ductal breast carcinomas with a size of 1 cm and larger could be detected at a concentration of 0.25 mg/kg body weight. Other fluorescent contrast agents, which can be used in near-infrared fluorescent contrast imaging are described in, for example, EP 1 113 822 A1 and Kai Licha *et al.* ((2000) *Photochemistry and Photobiology* **72**: 392-398).

For any imaging method administered in repeat screenings it is desired that they have as little side effects as possible. If the imaging technique requires the administration of substances like contrast agents it is desirable that only small amounts of such a substances are administered to avoid potential hazardous side effects and accumulation of the drug which might occur upon repeat administration. Thus, there is a need in the prior art to identify contrast agents which can be administered in small amounts and which will still provide the desired specificity and sensitivity for routine screening applications of DOT.

### Detailed Description of the Invention

Before the present invention is described in detail below, it is to be understood that this invention is not limited to the particular methodology, protocols, cell lines, vectors, and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Klbl, H. eds. (1995), Helvetica Chimica Acta, CH-4010 Basel, Switzerland).

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step.

Several documents are cited throughout the text of this specification. Each of the documents cited herein (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention.

It must be noted that as used herein and in the appended claims, the singular forms "a", "an", and "the", include plural referents unless the context clearly indicates otherwise. Thus, for example, reference to "a reagent" includes one or more of such different reagents, and reference to "the method" includes reference to equivalent steps and methods known to those of ordinary skill in the art that could be modified or substituted for the methods described herein.

Given above described need in the prior art it has now been surprisingly found by the present inventors that the previously known compound according to formula (I) 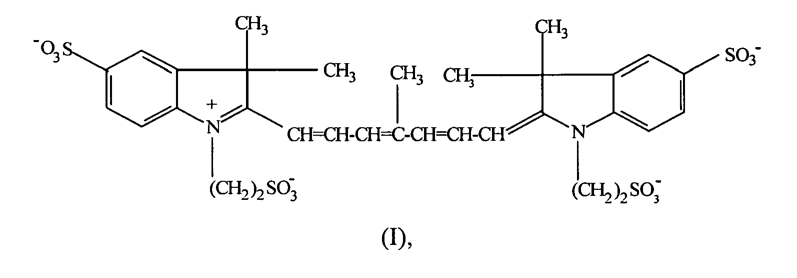 which is also known by the name SF64, provides a signal with a specificity and sensitivity sufficient for routine screening for proliferative diseases in amounts per kg body weight which are significantly lower than amounts per kg body weight which have been used in the prior art to obtain sufficient specificity and sensitivity. Thus, SF64 surprisingly can be administered in very small amounts and consequently with a low potential for toxic side effects. In addition the tumors detectable at this low concentration are as small as 3 mm in diameter and such significantly smaller than the tumors previously detected using other cyanine dyes like, e.g. ICG, for which detection limit are tumors of a size of 1 cm, i.e. comparable to x-ray mammography. The ability to detect tumors as small as 3 mm represents a significant advancement over the ability to detect tumors down to a size of 10 mm and has tremendous implications for the long term survival of the diagnosed patient. Tumors with a diameter of 1 cm or more have already attracted endothelial cells to form new capillaries, i.e. have been neo-vasularized, and have often already released tumor cells into the blood or lymph circulation. However, tumors with a diameter of 3 mm or less are often not vascularized and have stopped further growth due to a lack of nutrients. Since vascularization of the tumor is a prerequisite for further growth these tumors will only advance, if they develop the capability to attract endothelial cells. Consequently, the chances of preventing a cancer from ever developing into a life threatening disease is much higher, if the tumor can be detected already at a size where it is much less likely to have spread through the body and/or have attracted endothelial cells to form new capillaries.

Accordingly, a first aspect of the invention is the use of a compound according to formula (I) 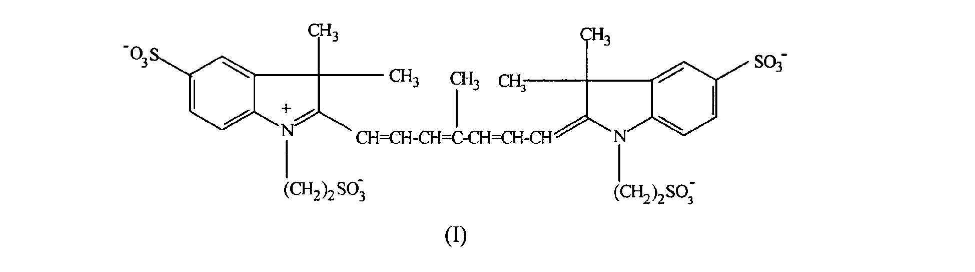 or a pharmaceutically acceptable salt thereof, for the preparation of a diagnostic composition for the detection of a proliferative disease, wherein the diagnostic composition comprises the compound in an amount of less than 0.5 and more than 0.001 mg/kg body weight per diagnostic application.

The ability of hydrophilic SF64 cyanine dye to act as contrast agent is in part determined by oxy- and dioxy-hemoglobin concentrations, blood oxidant saturation, contrast agent uptake into tissue and organical concentration, however, it is possible to increase the specificity and/or sensitivity of SF64 by coupling it to a targeting compound which binds specifically to structures which are preferentially or exclusively present on proliferating cells and tissues or in the vicinity of proliferating cells and tissue. Some of these structures are associated directly with the proliferating cell or are associated with cells in the vicinity of the proliferative tissue. The former are structures altered or over-expressed in the proliferating cell like, for example, growth factor receptors, like somatostatin receptor or epidermal growth factor receptor (FGFR). A large variety of such structures have been identified by now and comprise without limitation growth factor receptors, G-protein coupled receptors, pore proteins, ion channels, drug efflux pumps, accessory binding sites for growth factors, heparan sulfate, membrane bound proteases, adhesion molecules, T cell receptors and selectins, in particular EGF, TGF, CEA, Lewis Y, CD 20, CD 33, or CD38. Other structures, which can be targeted are T-cell-defined cancer-associated antigens belonging to unique gene products of mutated or recombined cellular genes, in particular cyclin-dependent kinase 4 (CDK4), p15^{Ink4b}, p53, AFP, β-catenin, caspase 8, p53, p21^{Ras} mutations, Bcr-abl fusion product, MUM-1 MUM-2, MUM-3, ELF2M, HSP70-2M, HST-2, KIAA0205, RAGE, myosin/m, 707-AP, CDC27/m, ETV6/AML, TEL/Aml1, Dekcain, LDLR/FUT, Pml-RARα, TEL/AMLI; Cancer-testis (CT) antigens, in particular NY-ESO-1, members of the MAGE-family (MAGE-A1, MAGE-A2, MAGE-A3, MAGE-A4, MAGE-A6 MAGE-10, MAGE-12), BAGE, DAM-6, DAM-10, members of the GAGE-family (GAGE-1, GAGE-2, GAGE-3, GAGE-4, GAGE-5, GAGE-6, GAGE-7B, GAGE-8), NA-88A, CAG-3, RCC-associated antigen G250; Tumor virus antigens, in particular human papilloma virus (HPV)-derived E6 or E7 oncoproteins, Epstein Barr virus EBNA2-6, LMP-1, LMP-2; overexpressed or tissue-specific differentiation antigens, in particular gp77, gp100, MART-1/Melan-A, p53, tyrosinase, tyrosinase-related protein (TRP-1 and TPR-2), PSA, PSM, MC1R; widely expressed antigens, in particular ART4, CAMEL, CEA, CypB, HER2/neu, hTERT, hTRT, ICE, Muc1, Muc2, PRAME RU1, RU2, SART-1, SART-2, SART-3, and WT1.

It is known that proliferating cells in particular tumor cells produce diffusible factors, which attract endothelial cells and stimulate them to grow. Therefore, tumors are one of the few areas within the body wherein new vascularization is observed. Consequently, the proliferating tumor endothelium and structures associated with it has been used to specifically target drugs to the tumor site. Molecular structures associated with angiogenesis are reviewed in, for example, WO 96/01653, Alessi P. *et al.* (2004) and Nanda, H. and Saint-Croix (2004). Cells which form proliferative tissues express both angiogenic and anti-angiogenic factors, which as long as angiogenesis inhibitors counteract the effect of the angiogenic factors leads to a suppression of angiogenesis. Once the effect of the angiogenic factors prevail they lead to initiation of angiogenesis. Thus, both structures, i.e. angiogenesis activators and inhibitors, which are involved in the regulation of angiogenesis can be bound by the targeting compound of the present invention. Angiogenesis activators include without limitation molecular structures like, e.g. ED-B fibronectin (ED-BF), endogline (CD105) (Burrows, F. J. *et al.* (1997) Clin. Cancer Res. 1: 1623-1634), VEGF-family members, vascular endothelial growth factor (VEGFR), NRP-1, Angl, Thie2, PDGF-bb and receptors, TGF-β1, TGF-β-receptors, FGF, HGF, MCP-1, integrants (αᵥβ₃, αᵥβ₅, α₅β₁), VE-cadherin, PICAM (CD31), ephrins, plasminogen activators, MMPs PAI-1, NOS, COX-2, A733, chemokines or Id1/Id Angiogenesis inhibitors include without limitation molecular structures like, e.g. VGFR-1, Ang2, TSP-1, -2, angiostatin and related plasminogen kringles, endostatin (collagene (XVII-fragment), vasostatin, platelet factor IV, TIMPs, MMP inhibitors, PEX, METH-1, METH-2, IFN-α, -β, -γ, IP-10, IL-4, IL-12, IL-18, prolactin, VEGI, fragment of SPARC, osteopontin fragment or maspin (Carmeliet, P. and Jain, R.K. (2000) *Nature* **407**: 249-257; Yancopoulos, G. D. *et al.* (2000) *Nature* **407**: 242-248; Bergers, G. and Benjamin, L.E. (2002) *Nature Reviews Cancer* 3: 401-410; Hendriks, M.J.C. *et al.* (2002) *Nature Reviews Cancer* **3**: 411-421).

In a preferred embodiment the targeting compounds bind to the angiogenesis specific factors ED-BF, VEGFR or endoglin. Out of those ED-BF is a particular preferred target structure. ED-BF is splice variant of fibronectin also called oncofoetal fibronectin, which is specifically formed in newly grown microvascular structures during angiogenesis.

The component that binds to these structures is preferably a peptide (amino acid chain with two to 50 amino acid residues), a protein (amino acid chains with more than 50 amino acid residues), a nucleic acid, a small molecule, or a sugar. In the remainder of this specification peptides and proteins are also commonly referred to as polypeptides.

Preferred polypeptides are ligands of structures, which are preferentially or exclusively expressed in proliferating cells or on the vicinity of proliferating cells like in vascularized or vascularizing structures, in particular vascular endothelial growth factor (VEGF), somatostatin, somatostatin analogues, bombesin, bombesin analogues, Vasoactive intestinal peptide (VIP) and analogues, neurotensin and neurotensin analogues, Neuropeptide Y and analogues and antibodies, including human, humanized and chimeric antibodies; antibody binding domain comprising fragments, e.g. Fv, Fab, Fab', F(ab')₂, Fabc, Facb; single chain antibodies, e.g. single chain Fvs (scFvs); and diabodies.

A large variety of such antibodies has been described in the literature and include for ED-BF: L19 and E8 (see Viti F. et al. (1999) Cancer Res. 59:347-352), the BC-1 monoclonal antibody described in EP 0 344 134 B1, which is obtainable from the hybridoma deposited at the European Collection of Animal Cell Cultures, Porton Down, Salisbury, UK under the number 88042101 or a chimeric or humanized version thereof, the antibodies against ED-BF with the specific V_{L} and V_{H} sequences disclosed in WO 97/45544 A1, the antibodies against ED-BF with the specific V_{L} and V_{H} sequences disclosed in WO 99/5857 A2, the antibodies against ED-BF with the specific V_{L} and V_{H} sequences disclosed in WO 01/62800 A1 and AP38 and AP39 (Marty C, et al. (2001) Protein Expr. Purif 21:156-64). Antibodies specific to ED-BF have been reviewed in Ebbinghaus C, et al. (2004) Curr Pharm Des. 10:1537-49. All these antibodies or antibody binding fragments thereof can be used as angiogenesis specific binding component in a preferred use of the present invention. Particularly preferred antibodies are L19, E8, AP 38 and AP 39 or binding domain comprising fragments thereof.

Antibodies for VEGF-R include Bevacizumab (Avastin™, rhumAb-VEGF developed by Genentech and Roche), the anti-VEGFR-1 antibody mAb 6.12, the fully human anti-VEGFR-2 antibodies IMC-2C6 and IMC-1121, the fully human anti-VEGFR-3 mAb HF4-3C5 (all Imclone Systems Inc.), and KM-2550 (Kyowa Hakko Kogyo Co. Ltd.), an anti-VEGFR-1 antibody (Salgaller ML (2003) Current Opinion in Molecular Therapeutics 5(6):657-667). Antibodies for endoglin include: SN6h, SN6, SN6a, SN6j, P3D1, P4A4, 44G4, GRE, E-9, CLE-4, RMAC8, PN-E2, MAEND3, TEC4, TEC11, A11, 8E11. Clone SN6h has been used extensively to study expression of endoglin in different tumor entities by immunohistochemistry (Wikström P. et al. (2002) The Prostate 51:268-275; Li C. et al. (2003) Br. J. Cancer 88:1424-1431; Saad R.S. et al. (2004) Modem Pathol. 17: 197-203). Of the same SN6 series antibodies SN6, SN6a and SN6j have been described (She X. et al. (2004) Int. J. Cancer 108:251-257). For the antibody clones P3D1, P4A4, 44G4, GRE, E-9, CLE-4, RMAC8, PN-E2, MAEND3, TEC4, TEC11 the binding epitopes of endoglin have been determined (Pichuantes S. et al. (1997) Tissue antigens 50:265-276). For some of these antibodies and antibody clone A11 the differential expression of endoglin has been investigated on normal and tumor tissues of human origin (Duff S. E. et al. (2003) FASEB J. 17:984-992). WO 02/02614 discloses further endoglin specific antibodies, e.g. scFv C4. In one of the last publications on antibodies against CD105 the clone 8E11 was investigated for its prediction of metastatic risk in breast cancer patients by immunohistochemistry (Dales J.P. et al. (2004) Br. J. Cancer 90:1216-1221). All these antibodies or antibody binding fragments thereof can be used as angiogenesis specific binding component in a preferred use of the present invention.

In addition many antibodies or binding fragments thereof, which specifically bind to various tumor cells themselves have been described in the prior art and include without limitation antibodies against G-protein coupled receptors, pore proteins, ion channels, drug efflux pumps, accessory binding sites for growth factors, heparan sulfate, membrane bound proteases, adhesion molecules, T cell receptors and selectins, in particular EGF, TGF, CEA, Lewis Y, CD 20, CD 33, or CD38. Furthermore antibodies or binding fragments thereof, against T-cell-defined cancer-associated antigens belonging to unique gene products of mutated or recombined cellular genes can be used, in particular cyclin-dependent kinase 4 (CDK4), p15^{Ink4b}, p53, AFP, β-catenin, caspase 8, p53, p21^{Ras} mutations, Bcr-abl fusion product, MUM-1 MUM-2, MUM-3, ELF2M, HSP70-2M, HST-2, KIAA0205, RAGE, myosin/m, 707-AP, CDC27/m, ETV6/AML, TEL/Aml1, Dekcain, LDLR/FUT, Pml-RARα, TEL/AMLI; Cancer-testis (CT) antigens, in particular NY-ESO-1, members of the MAGE-family (MAGE-A1, MAGE-A2, MAGE-A3, MAGE-A4, MAGE-A6 MAGE-10, MAGE-12), BAGE, DAM-6, DAM-10, members of the GAGE-family (GAGE-1, GAGE-2, GAGE-3, GAGE-4, GAGE-5, GAGE-6, GAGE-7B, GAGE-8), NA-88A, CAG-3, RCC-associated antigen G250; Tumor virus antigens, in particular human papilloma virus (HPV)-derived E6 or E7 oncoproteins, Epstein Barr virus EBNA2-6, LMP-1, LMP-2; overexpressed or tissue-specific differentiation antigens, in particular gp77, gp100, MART-I/Melan-A, p53, tyrosinase, tyrosinase-related protein (TRP-1 and TPR-2), PSA, PSM, MC1R; widely expressed antigens, in particular ART4, CAMEL, CEA, CypB, HER2/neu, hTERT, hTRT, ICE, Muc1, Muc2, PRAME RU1, RU2, SART-1, SART-2, SART-3, and WT1.

It is well known in the art that nucleic acids can possess specific binding properties, thus, the targeting component can also be a nucleic acid. Preferably, such nucleic acids include DNA, RNA, aptamers, and PNA, wherein aptamers are particularly preferred. Methods to identify specifically binding aptamers are well known in the art and are described, for example, in WO 93/24508 A1, WO 94/08050 A1, WO 95/07364 A1, WO 96/27605 A1, and WO 96/34875 A1. The methods disclosed in these documents are hereby specifically referenced and can be used in the identification of aptamers, which specifically bind to proliferating tissue or to tissue in the vicinity of proliferating tissue like newly formed and/or proliferating endothelium. Particularly preferred targeting compounds for the use of the present invention are angiogenesis specific binding aptamers. Preferred aptamers employed in the use of the present invention specifically recognize ED-BF, endoglin or VEGFR.

With the advent of high throughput screening of small molecules, i.e. non peptidyl, non nucleic acid compounds, of a molecular weight lower than 1.000 g/mol, preferably lower than 500 g/mol, it has been possible to identify small molecules with particular binding properties. Such small molecules can equally be employed as a targeting component in the use of the present invention. A preferred small molecule is 2,2-diphenylethylamine, which has been identified to specifically bind to ED-BF (Scheuermann J. (2002) Isolation of binding molecules to the EDB domain of fibronectin, a marker of angiogenesis. Dissertation submitted to Swiss Federal Inst. of Technology, Zurich).

Above targeting compounds can be coupled to SF64 by a direct or indirect bond. In this context the term "direct bond" means a covalent bond to a residue of the targeting compound while the term "indirect bond" as used herein means that one or more additional chemical residues which are connected by a covalent or non-covalent bond are located between the SF64 dye and the targeting compound. These one or more additional chemical residues is also termed "spacer". A spacer can, e.g. provide a spatial separation between the targeting compound and the SF64 dye. Several methods of coupling the SF64 dye to a targeting compound as outlined above are known in the art and usually involve the introduction of reactive functionalities into the dye, synthesize the dye as a derivative with reactive functionalities and/or into the targeting compound, which are capable of forming covalent bonds. Such groups include, for example, thio, hydroxy, amino or carboxy groups. For SF64 the coupling can be affected at either of the indole groups and/or the methyl groups at the 3 position of the indole heterocycle or the at methyl group located in the middle of the heptatrien or can be affected at any of the carbone residues of the heptatrien. In addition the coupling between the targeting compound and SF64 can be affected at the alkyl-chains linked to the indole nitrogen. Preferably, the targeting compound is coupled to one of the methyl groups at the 3 position of the indole or to the 4-methyl group of the heptatrien.

The term "ligand of receptors" refers to polypeptides, which specifically bind to cell surface receptors, i.e. which are natural binding partners of the receptor. The interaction between the receptor and its ligand can have different consequences, on one hand it is possible that the ligand and receptor simply act as a tether between, for example, two cells or that the binding can lead to a conformational or functional change of the receptor, which in turn can result in e.g. activation of an enzymatic function of receptor or association of the receptor with new and/or different further components within the cell membrane, on the extra cellular site or at the cytoplasmic site of the cell membrane. In this context ligands can have an agonistic or antagonistic effect on receptor function. Receptor ligands within the meaning of the invention are also modified ligands, which might carry additional N- or C-terminal amino acids or wherein amino acids have been replaced without a significant decrease of binding activity to the receptor. In this context a decrease by more than 90% would be considered significant. Preferably, modified ligands show an increase in specific binding. Examples of such ligands of receptors are VEGF, somatostatin and analogues thereof, bombesin or and bombesin analogues, Vasoactive intestinal peptide (VIP) and analogues, neurotensin and neurotensin analogues, Neuropeptide Y and analogues thereof.

Preferably, the compound according to formula (I) above is used as a sodium salt, however, it is also possible to use other pharmaceutical acceptable salts in addition to or instead of sodium, i.e. replacing one or more of the sodium ions in the molecule with one or more pharmaceutically acceptable other organic or inorganic cation including other alkali metal ions like potassium, alkaline earth metals such as magnesium, calcium and the like, organic cations such as triethyl ammonium, tributyl ammonium, pyridinium and the like, salts of amino acids, such as lysine, arginine and the like. A particularly preferred sodium salt of the compound usable according to the present invention is depicted in formula (II)

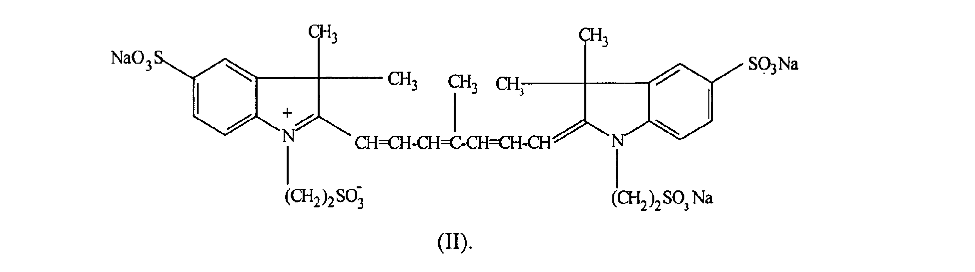

The proliferative diseases which can be diagnosed using above-indicated amount of the compound usable according to the present invention is preferably selected from the group consisting of a tumor, a precancerosis, a dysplasia, a metaplasia, psoriasis, psoriatic arthritis, rheumatoid arthritis, endometriosises and/or an ocular disease.

Preferred proliferative diseases, which are diagnosed, are tumors. Tumors can be further dif ferentiated in the primary tumor or a metastasis thereof. Often the cell which have metastasized grow more rapidly, shows less anchorage dependence, have a higher chromosomal ploidy and have gained the capability to extravasate from the circulation due to the expression of proteases like, e.g. collagenases, matrix metalloproteinases and the like. Thus, preferably a tumor is diagnosed prior to it having metastasized. While the risk that a primary tumor forms metastasizes in general increases with increasing size some tumors like, for example, melanomas often metastasise when the have a size of just one or a few millimetres other tumors are much less prone to early dissemination of tumor cells throughout the body like, for example, prostate cancer. A significant advantage of the use of the present invention is the ability to detect small sized proliferative lesions, in particular primary tumors. Preferably the prolif-erative lesion detected is smaller than 10 mm, preferably smaller than 9 mm, preferably smaller than 8 mm, preferably smaller than 7 mm, preferably smaller than 6 mm, preferably smaller than 5 mm and most preferably smaller than 4 mm.

Preferably the tumor diagnosed according to the use of the present invention is a malignoma of the gastrointestinal or colorectal tract, liver, pancreas, kidney, bladder, thyroid, prostate, endometrium, ovary, testes, melanoma, dysplastic oral mucosa, invasive oral cancer, small cell or non-small cell lung carcinoma; a mammary tumor, including hormone-dependent breast cancer, hormone independent breast cancers; transitional and squamous cell cancers; neurological malignancy including neuroblastoma, gliomas, astrocytomas, osteosarcoma, meningioma; soft tissue sarcoma; hemangioama and an endocrinological tumor, including pituitary adenoma, pheochromocytoma, paraganglioma, a haematological malignancy including lymphoma and leukemia or the metastasis originates from one of above mentioned tumors. Particularly preferred tumors are tumors of the breast, cervix, prostate, testis, wherein the organ/tissue from which the tumor developed is easily accessible from outside the body or by endoscopic means.

The precancerosis, which is detectable according to the use of the present invention is pref erably selected from the group consisting of precancerosis of the skin, in particular actinic keratosis, cutaneaous horn, actinic cheilitis, tar keratosis, arsenic keratosis, x-ray keratosis, Bowen's disease, bowenoid papulosis, lentigo maligna, lichen sclerosus, and lichen rubber mucosae; precancerosis of the digestive tract, in particular erythroplakia, leukoplakia, Barrett's esophagus, Plummer-Vinson syndrome, crural ulcer, gastropathia hypertrophica gigantea, borderline carcinoma, neoplastic intestinal polyp, rectal polyp, porcelain gallbladder; gynaecological precancerosis, in particular carcinoma ductale in situ (CDIS), cervical intraepithelial neoplasia (CIN), leukoplakia, endometrial hyperplasia (grade III), vulvar dystrophy, vulvar intraepithelial neoplasia (VIN), hydatidiform mole; urologic precancerosis, in particular bladder papillomatosis, Queyrat's erythroplasia, testicular intraepithelial neoplasia (TIN), leukoplakia; carcinoma in situ (CIS); precancerosis caused by chronic inflammation, in particular pyoderma, osteomyelitis, acne conglobata, lupus vulgaris, and fistula.

Dysplasia is frequently a forerunner of cancer, and is found mainly in the epithelia; it is the most disorderly form of non-neoplastic cell growth, involving a loss in individual cell uniformity and in the architectural orientation of cells. Dysplastic cells often have abnormally large, deeply stained nuclei, and exhibit pleomorphism. Dysplasia characteristically occurs where there exist chronic irritation or inflammation. Dysplastic disorders which can be diagnosed according to the present invention include, but are not limited to, anhidrotic ectodermal dysplasia, anterofacial dysplasia, asphyxiating thoracic dysplasia, atriodigital dysplasia, bronchopulmonary dysplasia, cerebral dysplasia, cervical dysplasia, chondroectodermal dysplasia, cleidocranial dysplasia, congenital ectodermal dysplasia, craniodiaphysial dysplasia, craniocarpotarsal dysplasia, craniometaphysial dysplasia, dentin dysplasia, diaphysial dysplasia, ectodermal dysplasia, enamel dysplasia, encephalo-ophthalmic dysplasia, dysplasia epiphysialis heminelia, dysplasia epiphysialis multiplex, dysplasia epiphysalis punctata, epithelial dysplasia, faciodigitogenital dysplasia, familial fibrous dysplasia of jaws, familial white folded dysplasia, fibromuscular dysplasia, fibrous dysplasia of bone, florid osseous dysplasia, hereditary renal-retinal dysplasia hidrotic ectodermal dysplasia, hypohidrotic ectodermal dysplasia, lymphopenic thymic dysplasia, mammary dysplasia, mandibulofacial dysplasia, metaphysical dysplasia, Mondini dysplasia, monostotic fibrous dysplasia, mucoepithelial dysplasia, multiple epiphysial dysplasia, oculoauriculovertebral dysplasia, oculodentodigital dysplasia, oculovertebral dysplasia, odontogenic dysplasia, ophthalmomandibulomelic dysplasia, periapical cemental dysplasia, polyostotic fibrous dysplasia, pseudoachondroplastic spondyloepiphysial dysplasia, retinal dysplasia, septo-optic dysplasia, spondyloepiphysial dysplasia, and ventriculoradial dysplasia.

Metaplasia is a form of controlled cell growth in which one type of adult or fully differentiated cell substitutes for another type of adult cell. Metaplastic disorders, which are detectable according to the use of the present invention are preferably selected from the group consisting of agnogenic myeloid metaplasia, apocrine metaplasia, atypical metaplasia, autoparenchymatous metaplasia, connective tissue metaplasia, epithelial metaplasia, intestinal metaplasia, metaplastic anemia, metaplastic ossification, metaplastic polyps, myeloid metaplasia, primary myeloid metaplasia, secondary myeloid metaplasia, squamous metaplasia, squamous metaplasia of amnion, symptomatic myeloid metaplasia and regenerative metaplasia.

The ocular disease, which is detectable according to the use of the present invention is preferably selected from the group consisting of trachoma, retinopathy of prematurity, diabetic retinopathy, neovascular glaucoma and age-related macular degeneration.

Endometriosis is a gynecological disease defined by the proliferation of endometrial tissue outside the uterine cavity. Proliferating endometrial cells can distribute through the entire body and endometrial lesions have already been found in the lung and in other organs and in that respect the distribution of endometrial lesions resembles the distribution of micrometastasis. In a preferred embodiment of the use of the present invention the endometric lesions, e.g. endometrial cell clusters, which are detected are hematogenous cell clusters, cavitary cell clusters, intraluminal cell clusters, lymphatic cell clusters, local cell clusters and/or regional cell clusters.

The use of the present invention can be for routine diagnosis i.e. for screening for the respectively indicated diseases. However, in a further embodiment the conjugates are used once the disease has been diagnosed with, for example, a standard x-ray procedure, e.g. mammography, a whole body scan or MRI. The patient is then examined for metastases and/or small (additional) primary tumor(s). Such an examination can occur for a better assessment of the severity, e.g. stage of the disease, or to determine the best treatment options and/or prior, during and/or after a treatment procedure (e.g. drugs, radiation or surgery). If performed prior to a treatment procedure the use of present invention due to its high sensitivity allows the determination whether, e.g. metastases have already formed in the vicinity of the primary tumor, and, thus, a better determination of the treatment regimen, e.g. whether a lumpectomy or rather a mastectomy is indicated in breast cancer.

After treatment the use of the diagnostic procedure of the present invention allows to assess the success of the treatment procedure and to determine subsequent treatment regiments, e.g. radiation or chemotherapy. When used during a surgical procedure it is, for example, possible to detect metastases in tissue, e.g. lymph nodes, surrounding of the primary tumor. In this embodiment the use of the present invention allows more complete removal of tumors or metastases during a surgical procedure.

In a further aspect of the use of the present invention the diagnostic composition further comprises pharmaceutical acceptable materials such as, for example, pharmaceutically acceptable salts to adjust the osmotic pressure, buffers, preservatives, carriers and/or excipients. Preferably the diagnositic composition is supplied in the form of a pyrogen-free parenterally acceptable pharmaceutical form, which may be an aqueous solution or a lyophilisate for the reconstitution prior to administration. The preparation of such a pharmaceutical composition having due regard to pH isotonicity, stability and the like is within the skill in the art. The diagnostic compositions used according to the present invention may include pharmaceutically acceptable diluents, such as, for example, sodium chloride injection and Ringer's injection. For administration to humans, the composition may be administered in autologous serum or plasma.

In a preferred use of the present invention the diagnostic compound is administered parenterally and more preferred intravenously. After intravenous administration of SF64 imaging *in vivo* can take place in a matter of a few minutes. However, imaging can take place, if desired hours or even longer after the SF64 has been injected to the patient. In most instances a sufficient amount of the administered dose will accumulate in the area to be imaged within about 0.1 hour. When using SF64 it has been surprisingly found that the contrast agent allowed the imaging of tumors up to 48 h after injection. This is an additional advantage over prior art dyes since it allows the administration of the compound usable according to the present invention one or event tow days prior to using DOT, for example, by the regular practitioner of a patient which then allows immediate imaging of the patient at a specialized screening facility without the need to inject the dye at the screening facility and to allow the distribution of the contrast agent at the day of carrying out DOT. Thus, in a preferred embodiment the diagnostic compound is administered at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 40, 41, 42, 43, 44, 45, 46, 47 or 48 h prior to imaging.

As outlined above it has been surprisingly found that much lower amounts of the cyanine dye SF64 as have been reported for other cyanine dyes like ICG are sufficient to provide the sensitivity and specificity required for detection of proliferative diseases. However, in a preferred embodiment of the use according to the present invention the compound according to formula (I) is comprised in the diagnostic composition in an amount of about 0.4, about 0.3, about 0.2, 0.1, less than 0.1, about 0.09, about 0.08, about 0.07, about 0.06, about 0.05, about 0.04, about 0.03, about 0.02, and about 0.01 mg/kg body weight. Consequently, preferred ranges of SF64 to be administered are between 0.4 and 0.001, between 0.2 and 0.001, between 0.1 and 0.001, between less than 0.1 and 0.001, between 0.09 and 0.001, between 0.008 and 0.001, between 0.06 and 0.001, between 0.05 and 0.001 mg per kg body weight. Due to the high hydrophilicity and high water solubility of SF64 injection volumes to the patient can be as low as 1 ml, which is significantly smaller than the currently used injections in excess of 10 ml. Therefore, in a preferred embodiment of the use according to the present invention the compound according to formula (I) is comprised in less than 5 ml, preferably less than 4 ml, preferably less than 3 ml, preferably less than 2 ml and even more preferably about 1 ml diagnostic composition.

A further aspect of the present invention is a diagnostic kit comprising the compound of formula (I) in an amount to prepare a diagnostic composition for administration of between less than 0.5 and 0.001 mg/kg body weight, preferably between 0.4 and 0.001, between 0.2 and 0.001, between 0.1 and 0.001, between less than 0.1 and 0.001, between 0.09 and 0.001, between 0.008 and 0.001, between 0.06 and 0.001, between 0.05 and 0.001 mg/kg body weight of the compound per diagnostic application and optionally a pharmaceutical acceptable salt, carrier, excipient and/or buffer as outlined above. Preferably, the kit comprises the compound and optional additional components in a lyophilized form.

Without further elaboration, it is believed that one skilled in the art can, using the preceding description, utilize the present invention to its fullest extent. The following preferred specific embodiments are, therefore, to be construed as merely illustrative, and not limitative of the disclosure in any way whatsoever.

### Brief Description of the Figures

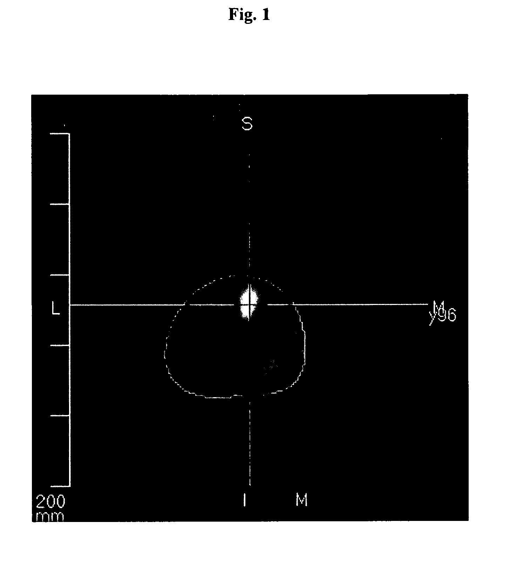

- **Fig. 1**: Optical mammography after i.v. injection of SF64 in a female patient with invasive breast cancer in the right breast. The bright spot is caused by a marked fluorescence signal of SF64, which after i. v. injection did accumulate in this invasive breast cancer.

### Example

### Optical mammography after i.v. injection of SF64 in a female patient with invasive breast cancer in the right breast.

A dose of 0.1 mg/kg body weight of SF64 was, after dilution of the lyophilisate with 0.9% normal saline, injected intravenously via an indwelling forearm canula. The patient was imaged with an optical computed tomographic laser mammography device in a prone position with her breast hanging freely into the imaging chamber, being surrounded by air. Image acquisition was started
1 hour 10 minutes after i.v. injection of SF64, and image reconstruction was performed using the fluorescence mode, thus almost exclusively showing the fluorescence signal of SF64. The contrast dye did accumulate in this invasive breast cancer, which is represented in the image by the bright spot. This led to the marked fluorescence signal of SF64 in the image.

## Claims

1. Use of compound according to formula (I) 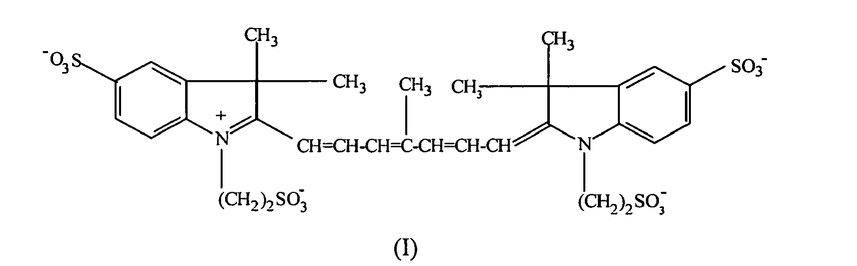 or a pharmaceutically acceptable salt thereof, for the preparation of a diagnostic composition for the detection of a proliferative disease, wherein the diagnostic composition comprises the compound in an amount of less than 0.5 and more than 0.001 mg/kg body weight per diagnostic application.

2. Use according to claim 1, wherein the compound is coupled to a targeting compound.

3. Use according to claim 2, wherein the targeting compound is selected from the group of targeting compounds consisting of a polypeptide, a nucleic acid, a small molecule or a sugar.

4. Use according to any of claim 1 to 3, wherein the polypeptide is selected from the group consisting of a receptor ligand, an antibody, a single chain antibody or a binding fragment of an antibody or single chain antibody

5. Use according to any of claims 1 to 4, wherein the proliferative disease is selected from the group consisting of a tumor, a precancerosis, a dysplasia, a metaplasia, psoriasis, psoriatic arthritis, rheumatoid arthritis, endometriosis and/or an ocular disease.

6. Use according to claim 5, wherein the tumor is a primary tumor or a metastasis.

7. Use according to claim 6, wherein the tumor is a malignoma of the gastrointestinal or colorectal tract, liver, pancreas, kidney, bladder, thyroid, prostate, endometrium, ovary, testes, melanoma, dysplastic oral mucosa, invasive oral cancer, small cell or non-small cell lung carcinoma; a mammary tumor, including hormone-dependent breast cancer, hormone independent breast cancers; transitional and squamous cell cancers; neurological malignancy including neuroblastoma, gliomas, astrocytomas, osteosarcoma, meningioma; soft tissue sarcoma; hemangioama and an endocrinological tumor, including pituitary adenoma, pheochromocytoma, paraganglioma, a haematological malignancy including lymphoma and leukemia or the metastasis originates from one of above mentioned tumors.

8. Use according to claim 6, wherein the diagnostic compound is administered during tumor screening or prior, during or after surgery.

9. Use according to claim 5, wherein the precancerosis is selected from the group consisting of precancerosis of the skin, in particular actinic keratosis, cutaneaous horn, actinic cheilitis, tar keratosis, arsenic keratosis, x-ray keratosis, Bowen's disease, bowenoid papulosis, lentigo maligna, lichen sclerosus, and lichen rubber mucosae; precancerosis of the digestive tract, in particular erythroplakia, leukoplakia, Barrett's esophagus, Plummer-Vinson syndrome, crural ulcer, gastropathia hypertrophica gigantea, borderline carcinoma, neoplastic intestinal polyp, rectal polyp, porcelain gallbladder; gynaecological precancerosis, in particular carcinoma ductale in situ (CDIS), cervical intraepithelial neoplasia (CIN), leukoplakia, endometrial hyperplasia (grade III), vulvar dystrophy, vulvar intraepithelial neoplasia (VIN), hydatidiform mole; urologic precancerosis, in particular bladder papillomatosis, Queyrat's erythroplasia, testicular intraepithelial neoplasia (TIN), leukoplakia; carcinoma in situ (CIS); precancerosis caused by chronic inflammation, in particular pyoderma, osteomyelitis, acne conglobata, lupus vulgaris, and fistula.

10. Use according claim 5, wherein the metaplasia is selected from the group consisting of agnogenic myeloid metaplasia, apocrine metaplasia, atypical metaplasia, autoparenchymatous metaplasia, connective tissue metaplasia, epithelial metaplasia, intestinal metaplasia, metaplastic anemia, metaplastic ossification, metaplastic polyps, myeloid metaplasia, primary myeloid metaplasia, secondary myeloid metaplasia, squamous metaplasia, squamous metaplasia of amnion, symptomatic myeloid metaplasia and regenerative metaplasia.

11. Use according to claim 5, wherein the dysplasia is selected from the group consisting of anhidrotic ectodermal dysplasia, anterofacial dysplasia, asphyxiating thoracic dysplasia, atriodigital dysplasia, bronchopulmonary dysplasia, cerebral dysplasia, cervical dysplasia, chondroectodermal dysplasia, cleidocranial dysplasia, congenital ectodermal dysplasia, craniodiaphysial dysplasia, craniocarpotarsal dysplasia, craniometaphysial dysplasia, dentin dysplasia, diaphysial dysplasia, ectodermal dysplasia, enamel dysplasia, encephalo-ophthalmic dysplasia, dysplasia epiphysialis heminelia, dysplasia epiphysialis multiplex, dysplasia epiphysalis punctata, epithelial dysplasia, faciodigitogenital dysplasia, familial fibrous dysplasia of jaws, familial white folded dysplasia, fibromuscular dysplasia, fibrous dysplasia of bone, florid osseous dysplasia, hereditary renal-retinal dysplasia hidrotic ectodermal dysplasia, hypohidrotic ectodermal dysplasia, lymphopenic thymic dysplasia, mammary dysplasia, mandibulofacial dysplasia, metaphysical dysplasia, Mondini dysplasia, monostotic fibrous dysplasia, mucoepithelial dysplasia, multiple epiphysial dysplasia, oculoauriculovertebral dysplasia, oculodentodigital dysplasia, oculovertebral dysplasia, odontogenic dysplasia, ophthalmomandibulomelic dysplasia, periapical cemental dysplasia, polyostotic fibrous dysplasia, pseudoachondroplastic spondyloepiphysial dysplasia, retinal dysplasia, septo-optic dysplasia, spondyloepiphysial dysplasia, and ventriculoradial dysplasia.

12. Use according to claim 5, wherein the ocular disease is selected from the group consisting of trachoma, retinopathy of prematurity, diabetic retinopathy, neovascular glaucoma and age-related macular degeneration.

13. Use according to any of claims 1 to 12, wherein the diagnostic composition further comprises a pharmaceutically acceptable salt, carrier, excipient and/or buffer.

14. Use according to any of claims 1 to 13, wherein the compound is comprised in the diagnostic composition in an amount of 0.1 or less mg/kg body weight.

15. A diagnostic kit comprising the compound of formula (I) according to claim 1 or a pharmaceutically acceptable salt thereof in an amount to prepare a diagnostic composition for administration of less than 0.5 and more than 0.001 mg/kg body weight of the compound per diagnostic application and optionally a pharmaceutically acceptable salt, carrier, excipient and/or buffer.
